# EUROPEAN PATENT APPLICATION

(11) **EP 4 180 033 A1**
(43) Date of publication of application: **17.05.2023**
(21) Application number: 21837895.8
(22) Date of filing: 08.07.2021
(51) Int. Cl.: A61K 31/165, A61K 31/138, A61K 31/517, A61K 31/5377, A61P 17/02, A61P 17/00, A61P 35/00, A61P 1/04, A61P 27/02

(54) **USE OF ß-1 ADRENOCEPTOR ANTAGONIST FOR PREPARING COMPOSITION FOR REDUCING EPITHELIAL CELL DAMAGE INDUCED BY EPIDERMAL GROWTH FACTOR RECEPTOR INHIBITOR AND INHIBITING CANCER CELLS**

(30) Priority: 10.07.2020 US 202063050390 P
(71) Applicant: Chang Gung Memorial Hospital, Linkou, Taoyuan City 33305 (TW); Chang Gung University, Taoyuan County 33302, Taiwan (TW)
(72) Inventor: LU, Chun-Wei, Fuxin, Taiwan 33305 (CN); SU PANG, Jong-Hwei, Taiwan 33302 (CN); KO, Yu-Shien, Fuxin, Taiwan 33305 (CN); CHUNG, Wen-Hung, Fuxin, Taiwan 33305 (CN); CHEN, Mei-Jun, Fuxin, Taiwan 33305 (CN)
(74) Representative: Cabinet Becker et Associés
(86) International application number: PCT/CN2021/105167
(87) International publication number: WO 2022/007878

(57) **Abstract**

The present invention relates to the use of β-1 adrenergic receptor antagonist to prepare compositions for reducing or preventing damage to normal epithelial cells induced by epidermal growth factor receptor inhibitor (EGFRI) and inhibiting cancer cells, by administering a composition containing a β-1 adrenergic receptor antagonist to reduce damage to normal epithelial cells induced by the EGFRI. The composition comprising β-1 adrenergic receptor antagonist is administered to reduce damage to normal epithelial cells induced by the EGFRI. The composition comprising β-1 adrenergic receptor antagonist can be administered together with an epidermal growth factor receptor inhibitor to synergistically inhibit cancer cells.

## Description

### TECHNICAL FIELD

### Cross-references to related applications

This application claims the benefit of US Application No. 63/050,390, filed on July 10, 2020. The entire contents of which are incorporated herein by reference

The present invention relates to the use of a β-1adrenergic receptor antagonist for preparing a composition for reducing or preventing epidermal growth factor receptor inhibitor-induced damage to normal epithelial cells and inhibiting cancer cells.

### BACKGROUND

Epidermal Growth Factor Receptor (EGFR) is regarded as an effective target for the development of anti-tumor therapy. It has been more than 15 years since the first introduction of EGFR-targeted therapy, which has helped countless cancer patients to prolong their lives. Epidermal growth factor receptor inhibitor (EGFRI) includes anti-epidermal growth factor receptor monoclonal antibody (mAb) and epidermal growth factor receptor tyrosine kinase inhibitor (EGFR-TKI).

Amongst them, the mechanism of action of the anti-epidermal growth factor receptor monoclonal antibody is specific binding to the epidermal growth factor receptor, and competitively inhibit the function of the epidermal growth factor, so that the cancer cells cannot proliferate. Currently, the commonly used monoclonal antibody drugs in clinical setting are Cetuximab, Zalutumumab, Nimotuzumab, Matuzumab and Panitumumab.

EGFR-TKI inhibits the activity of tyrosine kinase. Because tyrosine kinase acts as a switch of many intracellular signal transmissions, it plays an important role in cell growth, proliferation and differentiation, and its mutation often causes cancer.

Therefore, tyrosine kinase inhibitors can be used as an anti-cancer drug. In addition to inhibiting the proliferation of cancer cells, they also prevent new angiogenesis and block the supply of nutrients and oxygen to cancer cells. Currently, the commonly used tyrosine kinase inhibitors in clinical setting include erlotinib, gefitinib, lapatinib, afatinib, dacomitinib, osimertinib, etc.

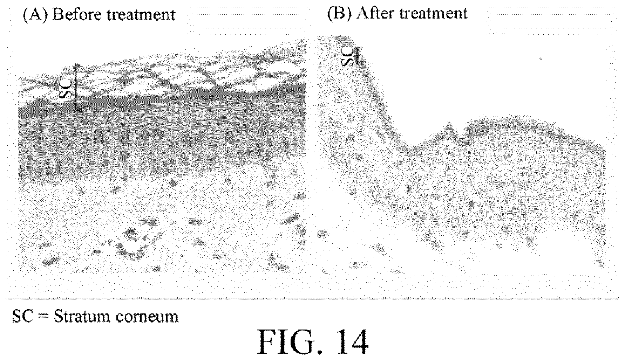

Although epidermal growth factor receptor inhibitors can be used to treat a variety of cancers, the following epithelial cell damages are still common in clinical practice, including: intestinal epithelial toxicity, lung toxicity, liver toxicity and epithelial cell damage of the skin. Skin epithelial cell damage includes papulopustular rash, purpuric drug eruption, skin thinning, dermatitis, rosacea, xerosis, hair thinning, curly hair, or skin barrier damage. Due to the high expression of EGFR in the epidermal base, the use of epidermal growth factor receptor inhibitor is often associated with cutaneous adverse drug reactions that damage normal or uninjured epithelial cells. Compared with normal skin, epidermal growth factor receptor inhibitor can cause inflammatory cell infiltration in the skin, especially in the hair follicle, thinning of the stratum corneum layer of the skin, the loss of normal basket-weave structure and a denser, more eosinophilic, and parakeratotic appearance of the stratum corneum layer (see FIG. 14, excerpted from Herbst, RS, et al., Dermatologic side effects associated with gefitinib therapy: clinical experience and management. Clin Lung Cancer, 2003. 4(6): p. 366-9). The current management of EGFRI induced cutaneous adverse drug reactions includes dose reduction or discontinuation of EGFRI therapy. Thus, there remains a need to reduce or prevent EGFRI induced epithelial cell damage. The present invention addresses this need and other needs.

### SUMMARY OF THE INVENTION

In view of the above problems, the object of the present invention is to provide the use of β-1 adrenergic receptor antagonist for manufacturing a composition for reducing or preventing EGFRI-induced damage to normal epithelial cells.

To address the damage caused by epidermal growth factor receptor inhibitor (EGFRI) for the treatment of cancer to normal (or not yet injured) epithelial cells, a composition comprising β-1 adrenergic receptor antagonist is administered to a subject or a patient in need thereof to reduce or prevent damage to normal epithelial cells caused by the administration of EGFRI for the treatment of cancer.

In one embodiment, said normal epithelial cells are epithelial cells that have not been damaged by an EGFRI or by the toxicity induced by an EGFRI.

In one example, said β-1 adrenergic receptor antagonist may be administered before, after, or concurrently with an EGRFI. In one example, the β-1 adrenergic receptor antagonist may be administered before the administration an EGFRI.

Some embodiments of the present invention are directed to methods of inhibiting normal (or not yet injured) epithelial cell damage in a subject due to the use of EGFRI for cancer treatment, comprising administering to the subject in need thereof an effective amount of at least one β-1 adrenergic receptor antagonist, thereby reducing or preventing the symptoms and/or signs of epithelial cell damage in the subject.

Some specific examples of the present invention provide the use of a β-1 adrenergic receptor antagonist for manufacturing a composition for inhibiting cancer cells. The composition comprises a combination of at least one β-1 adrenergic receptor antagonist and at least one EGRFI. The combination of at least one β-1 adrenergic receptor antagonists and at least one EGFRI can produce additive or synergistic effects.

Some specific examples of the present invention are directed to methods of inhibiting cancer growth in a subject comprising administering to the subject in need thereof an effective amount of a combination including at least one β-1adrenergic receptor antagonist and at least one EGFRI, thereby alleviating the symptoms and/or signs of cancer in the subject.

The compositions and methods of the present invention can be used to treat or inhibit the growth of any type of cancer. In some specific examples, the cancer to be treated or the growth of cancer to be inhibited is a solid tumor or hematological tumor, such as liver cancer, bile duct cancer, breast cancer, lung cancer, stomach cancer, pancreatic cancer, colorectal cancer, uterine cancer, cervical cancer, leukemia and lymphoma.

In one embodiment, said EGFRI is an epidermal growth factor receptor tyrosine kinase inhibitor (EGFR-TKI). Non-limiting examples of EGFR-TKI include erlotinib, gefitinib, lapatinib, afatinib, dacomitinib, osimertinib, or any combination thereof.

In one embodiment, said EGFRI is an anti-epidermal growth factor receptor (EGFR) monoclonal antibody. Non-limiting examples of anti-EGFR monoclonal antibody include Erlotinib, Gefitinib, Lapatinib, Afatinib, Dacomitinib, Osimertinib, or any combination thereof.

In one embodiment, said epithelial cells refer to skin epithelial cells, intestinal epithelial cells or corneal epithelial cells.

In one embodiment, non-limiting examples of the β-1 adrenergic receptor antagonists include atenolol, betaxaolol, bisoprolol, esmolol, acebutolol, metoprolol, nebivolol, or any combination thereof.

In one embodiment, the EGFRI-induced skin epithelial cytotoxicity comprises at least one of the following: papulopustular rash, purpuric drug eruption, skin thinning, dermatitis, rosacea, dryness, hair thinning, curly hair or skin barrier damage.

In one embodiment, the EGFRI-induced intestinal epithelial cell toxicity comprises at least one of the following: oral mucositis, genital mucositis, or diarrhea.

In one embodiment, the composition comprising a β-1 adrenergic receptor antagonist may further comprise the aforementioned EGFRI, said composition is used to inhibit the growth of cancer cells and reduce EGFRI induced epithelial cell damage.

The terms "invention," "the invention," "this invention," and "the present invention" as used in herein are intended to broadly refer to the subject matter of the invention and the full scope of the following claims. Statements containing these terms should be understood not to limit the meaning of, or the scope of, the subject matter of the invention described herein or the scope of the claims below. The embodiments of the invention covered by the present application are defined by the following claims, not by the content of the present invention. The present application is a high-level overview of various aspects of the invention and introduces some concepts that are further described in the following description. The present application is not intended to identify key or essential features of the claimed subject matter, nor is it intended to be used alone to determine the claimed subject matter. The subject matter of the invention should be understood by reference to the entire specification, any or all drawings, and appropriate portions of the scope of each claim.

The invention will become more apparent when read in conjunction with the accompanying drawings and the following detailed description.

### BRIEF DESCRIPTION OF DRAWINGS

FIG. 1 is a schematic diagram showing the cell viability of epithelial cells after contacting various concentrations of EGFR-TKI with normal epithelial cells.
FIG. 2 is a schematic diagram showing the epithelial cell viability after contacting various concentrations of β-1 adrenergic receptor antagonist (betaxolol) with normal epithelial cells.
FIG. 3 is a schematic diagram showing the epithelial cell viability, after contacting the normal epithelial cells with a β-1 adrenergic receptor antagonist (betaxolol), followed by contacting an EGFR-TKI one hour later.
FIG. 4 is a schematic diagram showing the epithelial cell viability after simultaneously contacting the normal epithelial cells with an EGFR-TKI and a β-1 adrenergic receptor antagonist.
FIG. 5 is a schematic diagram showing the A549 cancer cell viability after contacting the cancer cells with an EGRF-TKI.
FIG. 6 is a schematic diagram showing the of A549 cancer cell viability after contacting the cancer cells with an EGFR-TKI only or simultaneously contacting the cancer cells with an EGFR-TKI and a β-1 adrenergic receptor antagonist.
FIG. 7 is a schematic diagram showing the PC9 cancer cell viability after contacting the cancer cells with an EGFR-TKI only or simultaneously contacting the cancer cells with an EGFR-TKI and a β-1 adrenergic receptor antagonist.
FIG. 8 is a schematic diagram showing epithelial cell viability after contacting the normal epithelial cells with a non-selective β-adrenergic receptor antagonist, followed by contacting an EGFR-TKI one hour later.
FIG. 9 is a schematic diagram showing the A549 cancer cell viability after contacting the cancer cells with an EGFR-TKI and a non-selective β-adrenergic receptor antagonist.
FIG. 10 to FIG. 12 are schematic diagrams showing the effect of β-1 adrenergic receptor antagonist on EGFR-TKI-induced rosacea.
FIG. 13 illustrates the effect of β-1 adrenergic receptor antagonists on EGFR monoclonal antibody-induced papulopustular rash.
FIG. 14 is the eosin-stained image of normal skin cells (Panel A) and skin cells after contacting an EGFRI (Panel B) (excerpted from Herbst, RS, et al., Dermatologic side effects associated with gefitinib therapy: clinical experience and management. Clin Lung Cancer, 2003. 4(6): p.366-9).
FIG. 15 shows the epithelial cells viability after contacting a normal epithelial cells with a β-1 adrenergic receptor antagonist (bisoprolol), followed by contacting an EGFR-TKI one hour later.
FIG. 16 shows the epithelial cell viability after contacting normal epithelial cells with a β-1 adrenergic receptor antagonist (acebutolol), followed by contacting an EGFR-TKI one hour later.

### IMPLEMENTATION

The following description is accompanied with the drawings and specific working examples to illustrate the technical content of the present invention. Those skilled in the art can easily understand other advantages and effects of the present invention from the disclosure of this specification. The present invention can also be implemented or applied through other working examples. Various details in this specification can also be modified and changed based on different viewpoints and applications without departing from the spirit of the present invention.

The terms "subject" and "patient" are used interchangeably and refer to a mammal diagnosed with or suspected of having EGFRI-induced epithelial cell damage and/or cancer. Subjects include primates, preferably humans.

An "effective amount" of an antagonist is an amount of an inhibitor that produces a desired effect, for example, a reduction in the rate of epithelial cell damage and/or rate of cancer cell inhibition by at least about 1%, 5%, 10%, 20%, 30%, 40%, 50%, 60%, 70%, 80%, 90% or 95%, as compared to an untreated subject,

The technical term "treatment" as used herein includes preventative (for example, prophylactic), palliative and curative uses or results. Subjects or patients in need of treatment or prevention are those diagnosed with or suspected of having epithelial cell damage; subjects or patients are those diagnosed or suspected of having cancer, or patients whose normal epithelial cells are not yet damaged by EGFRI.

All numbers herein may be understood as modified by "about." As used herein, the term "about" refers to a range of ±10% of a specified value.

In addition, one skilled in the art can quickly determine the dosage and dose numbers of β-1 adrenergic receptor antagonists for a particular type of epithelial cell damage, depending on, for example, the severity and type of epithelial cell damage, the patient's age, weight, gender, complications, and other co-administered drugs. Those skilled in the art will appreciate that the preferred dose is one that produces a therapeutic effect in a patient in need thereof, such as accelerating epithelial cell repair.

The β-1 adrenergic receptor antagonist can be administered in any effective amount. In some specific examples, the dose to be administered maybe within the range from about 0.01% w/w to about 10% w/w, from about 0.05% w/w to about 5% w/w, from about 0.1% w/w to about 1% w/w.

Suitable dosages of the compositions provided herein are determined by comparing the *in vitro* activity of the compositions provided herein with *in vivo* activity in animal models. Methods for extrapolating effective doses in mice and other animals to effective doses in human are known in the art; for example, US Pat. No. 4,938,949, which is incorporated herein by reference.

The composition is administered in an effective amount to inhibit epithelial cell damage or reduce cancer cell growth. The dosage of the pharmaceutical composition to be administered depends on the severity of the condition, the particular formulation, and other clinical factors (such as the recipient's weight, the general condition and route of administration). In one embodiment, a single dose is administered once a day for a given number of days (for example, 1 day, 7 days, 14 days, 21 days, 1 month, etc.).

In another embodiment, multiple doses may be administered in one day (every 2 hours, 4 hours, 6 hours, or 12 hours, etc.) or for multiple days.

According to the methods provided herein, the composition can be delivered by any of the following routes including, but not limited to, injection (for example, subcutaneous, intramuscular, intravenous, intra-arterial, intraperitoneal, intradermal); skin; dermis; transdermal; oral (for example, lozenges, pills, liquids, edible tapes); implanted osmotic pumps; suppositories; aerosol sprays; topical; intraarticular; ocular, nasal, inhalation, skin and vagina insertion.

In one embodiment, the composition is formulated for topical delivery in any of the following forms: ointment, cream, solution, gel, suspension, spray or lotion. In another embodiment, the composition is formulated as slow or sustained release.

### EXAMPLE 1

The MTT test (i.e., cell viability analysis) was performed to examine the effect of epidermal growth factor receptor tyrosine kinase inhibitor (EGFR-TKI) on epithelial cells. The steps are as follows:

A fixed number (7×10⁴ cells/dish) of HaCaT cells (human skin keratinocytes) were seeded in each well of a multi-well plate (24 wells). After 24 hours of culture, different concentrations of afatinib (0, 1, 2.5, 5, 10, 20, 50 µM) and 0.1% DMSO were added to the cells and incubated for 24 hours. MTT (3-(4,5-dimethylthiazol-2-yl)-2,5-diphenyltetrazolium bromide and 300λ bromide-3-(4,5-dimethyl-2-thiazole)-2,5-diphenyltetrazolium) were added and 1 hour later, the supernatant was removed, DMSO was added and the resultant was shook. 200λ/well of liquid was extracted and added into another multi-well plate (96 wells). ELISA was used to measure the absorbance value (OD570, optical density 570nm) and calculate the cell cell viability. The experiment was repeated 3 times. Table 1 below shows the results of absorbance value and cell viability.

**Table 1**

| Afatinib | 0µM | DMSO | 0.1µM | 0.5µM | 1µM | 2.5µM | 5µM |
|---|---|---|---|---|---|---|---|
| Absorbance value (OD₅₇₀) | 0.439 | 0.420 | 0.424 | 0.422 | 0.404 | 0.282 | 0.159 |
| | 0.441 | 0.441 | 0.386 | 0.380 | 0.375 | 0.278 | 0.149 |
| | 0.443 | 0.429 | 0.389 | 0.386 | 0.374 | 0.288 | 0.153 |
| Cell viability (%) | 100.0 | 97.5 | 90.6 | 89.8 | 87.2 | 64.1 | 34.8 |

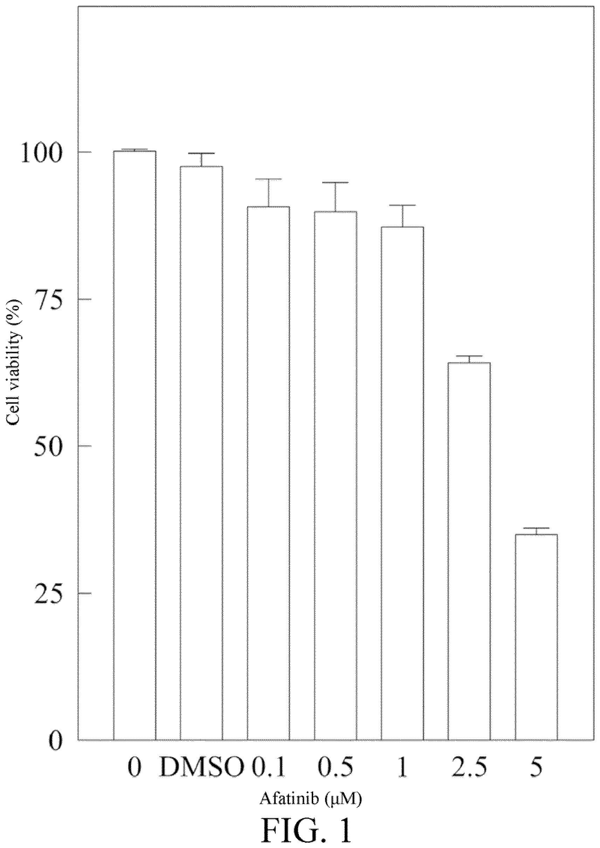

Referring to FIG. 1, FIG. 1 is a schematic diagram the epithelial cell viability after the normal epithelial cells contacted various concentrations of EGFRI. According to the figure, the HaCaT cell viability gradually decreased as the concentration of afatinib increased. When the afatinib concentration increased to 5 µM, the cell viability was only 34.8%. Therefore, EGFR-TKI increases apoptosis of normal epithelial cells.

### Example 2

The MTT test was carried out to examine the effect of β-1 adrenergic receptor antagonists on epithelial cells. The experimental procedure was similar to that of Example 1 and not be repeated here, except that afatinib was replaced by betaxolol. The experiments were repeated 4 times and the data of absorbance and cell viability are shown in Table 2 below.

**Table 2**

| Betaxolol | 0µM | 10µM | 20µM | 40µM | 80µM | 100µM |
|---|---|---|---|---|---|---|
| Absorbance (OD₅₇₀) | 0.531 | 0.493 | 0.485 | 0.492 | 0.480 | 0.466 |
| | 0.539 | 0.498 | 0.492 | 0.478 | 0.477 | 0.477 |
| | 0.532 | 0.496 | 0.486 | 0.495 | 0.487 | 0.469 |
| | 0.530 | 0.498 | 0.488 | 0.489 | 0.479 | 0.472 |
| Cell viability (%) | 100.0 | 92.8 | 91.3 | 91.4 | 90.1 | 88.1 |

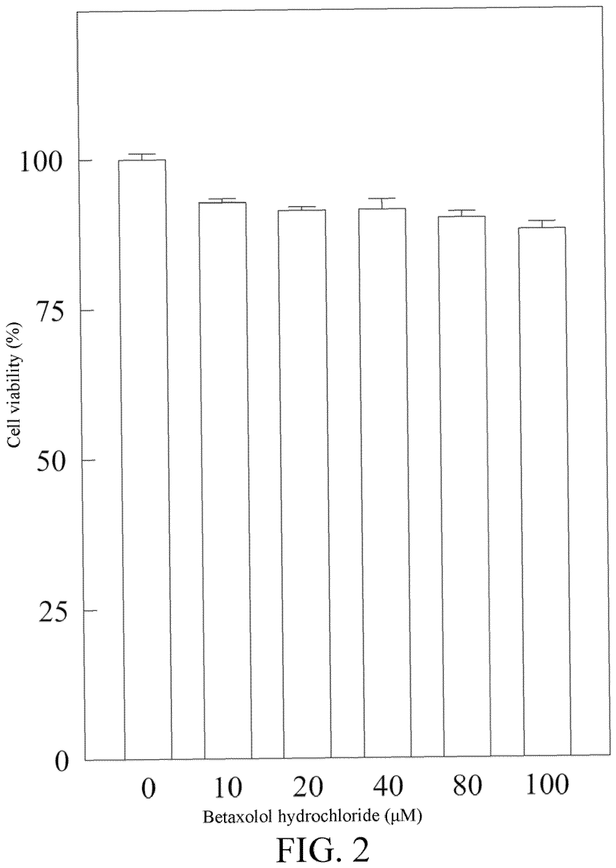

Referring to FIG. 2, FIG. 2 is a schematic diagram showing the epithelial cell viability after contacted the normal epithelial cells with various concentrations of β-1 adrenergic receptor antagonist (betaxolol). As illustrated by the figure, as the concentration of betaxolol increased, the cell viability of HaCaT cells was at least 90%. When the betaxolol concentration increased to 100 µM, the cell viability dropped to 88.1%. Therefore, betaxolol basically does not affect the cell viability of normal epithelial cells.

### EXAMPLE 3

The MTT test was carried out to examine the effect of first administering β-1 adrenergic receptor antagonist followed by EGFR-TKI on epithelial cells. The experimental procedure was similar to that of Example 1. HaCaT cells were contacted with betaxolol and an hour later, afatinib was added and the cells were cultured for 24 hours to observe the cell viability.

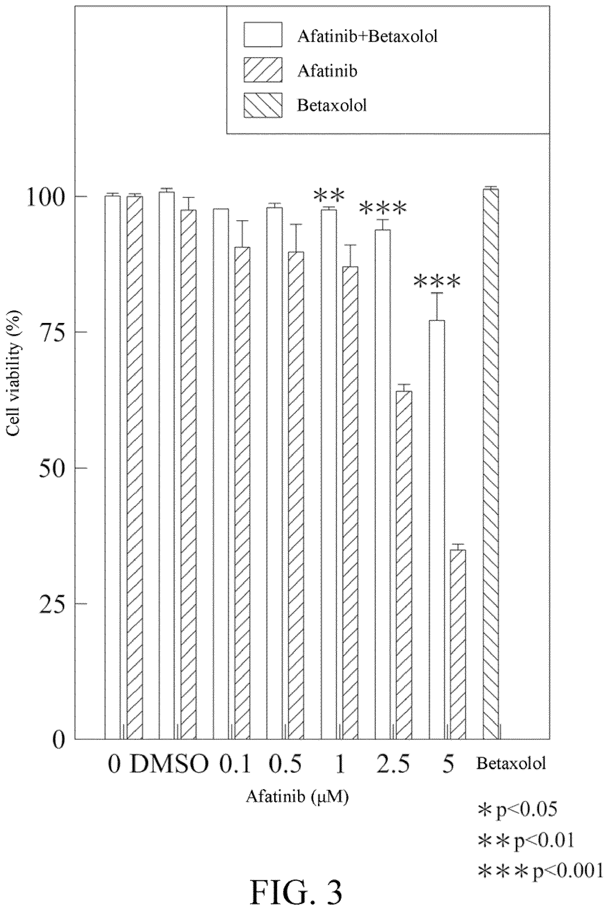

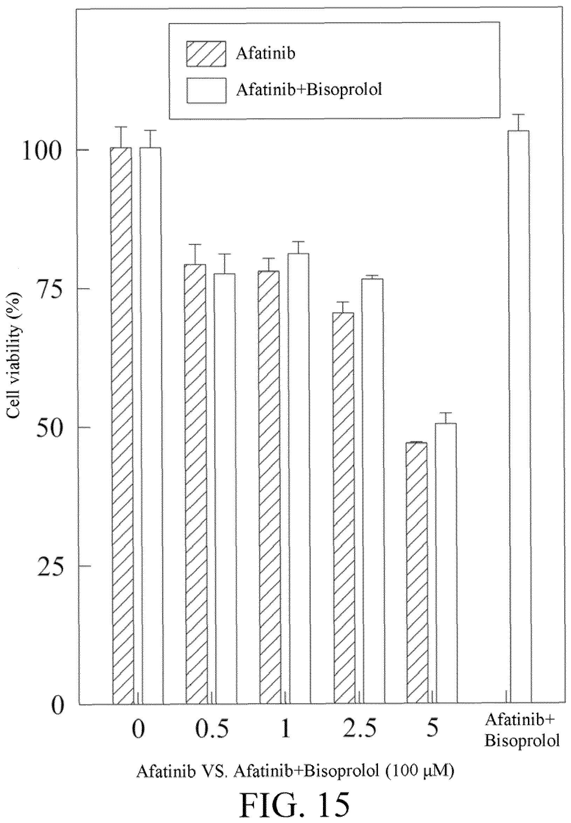

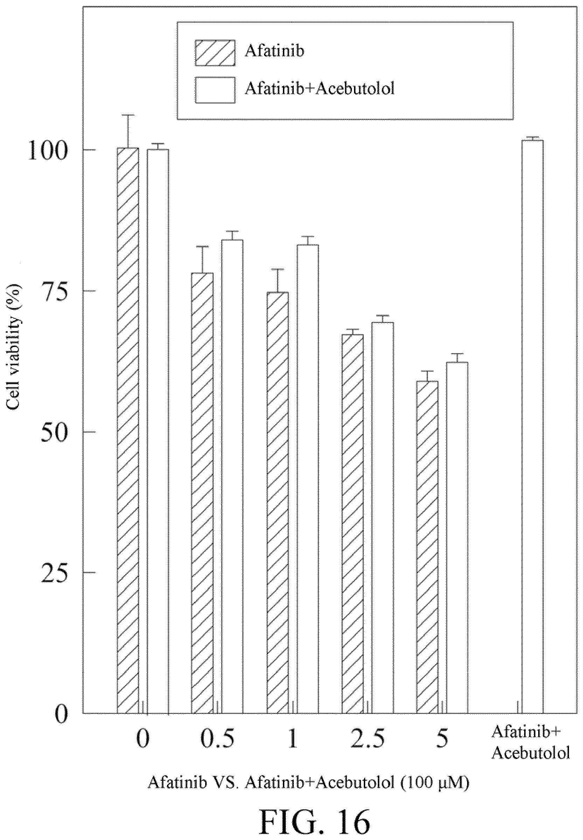

Referring to FIG.s 3, 15 and 16, these figures show the epithelial cell viability, as the normal epithelial cells first contacted a β-1adrenergic receptor antagonist (betaxolol), followed by adding an EGFR-TKI one hour later. According to FIG. 3, FIG. 15 and FIG. 16, the HaCaT cell viability was 100% if the cells were only in contact with a β-1 adrenergic receptor antagonist. The HaCaT cell viability of reduced in a dose dependent manner if the cells were only in contact with afatinib. When the HaCaT cells contacted the beta-1 adrenergic receptor antagonist first followed by afatinib, the cell viability of HaCaT cells was higher compared with the cell viability of HaCaT cells in contact with afatinib only. As the concentration of afatinib increased, the apoptosis of HaCaT cells was significantly reduced. Therefore, β-1 adrenergic receptor antagonists can indeed reduce the epithelial cell damage caused by EGFR-TKI.

### EXAMPLE 4

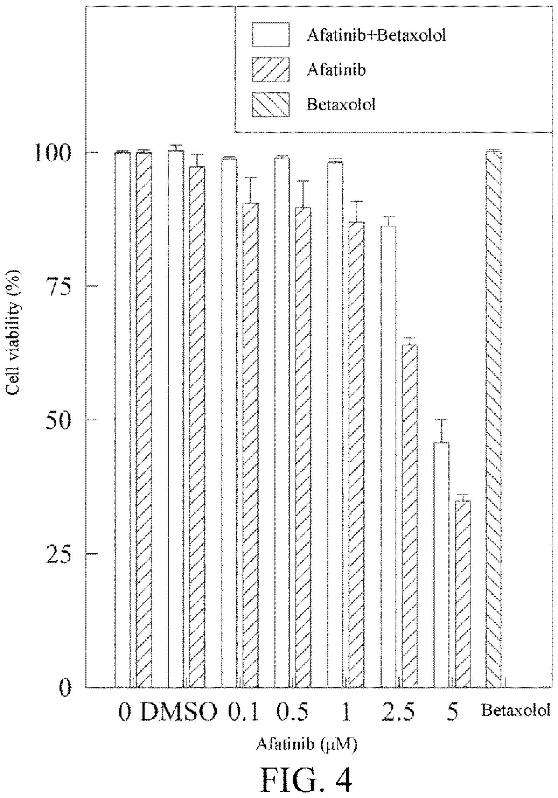

A MTT test was conducted to examine the effect of simultaneously administering a β-1 adrenergic receptor antagonist and an EGFR-TKI on epithelial cells. The experimental procedure was similar to that of Example 1. Betaxolol and afatinib were simultaneously added to HaCaT cells and the cells were cultured for 24 hours to observe cell viability.Referring to FIG. 4, FIG. 4 is a schematic diagram showing the c epithelial cell viability after simultaneously contacting an EGFR-TKI and a β-1 adrenergic receptor antagonist with normal epithelial cells. According to the figure, the cell viability of HaCaT cells was 100% after contacting with betaxolol. For HaCaT cell viability decreased as afatinib concentration increased for cells in contact with afatinib only. When betaxolol and afatinib were simultaneously added to HaCaT cells, the cell viability was much improved compared to the cell viability of HaCaT cells in contact with afatinib only. Accordingly, β-1 adrenergic receptor antagonists can indeed reduce the epithelial cell damage induced by EGFR-TKI.

### EXAMPLE 5

From the foregoing examples, it was found that the normal epithelial cell viability increases with simultaneous administration of a β-1 adrenergic receptor antagonist and an EGFR-TKI to normal epithelial cells.

The effect of EGFR-TKI alone and the simultaneous administration of a β-1 adrenergic receptor antagonist and an EGFR-TKI on cancer cells was examined. The experimental procedure was similar to that of Example 1, and details not repeated here.

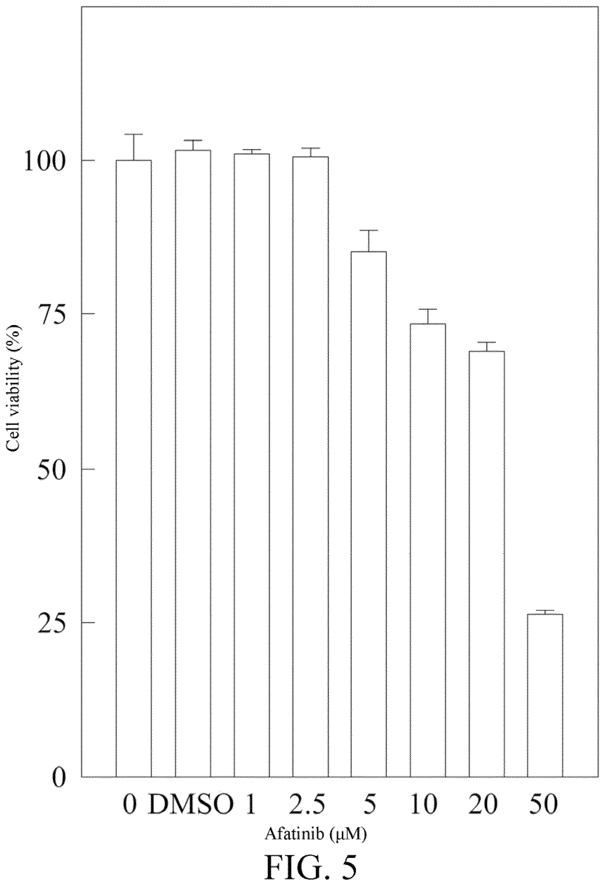

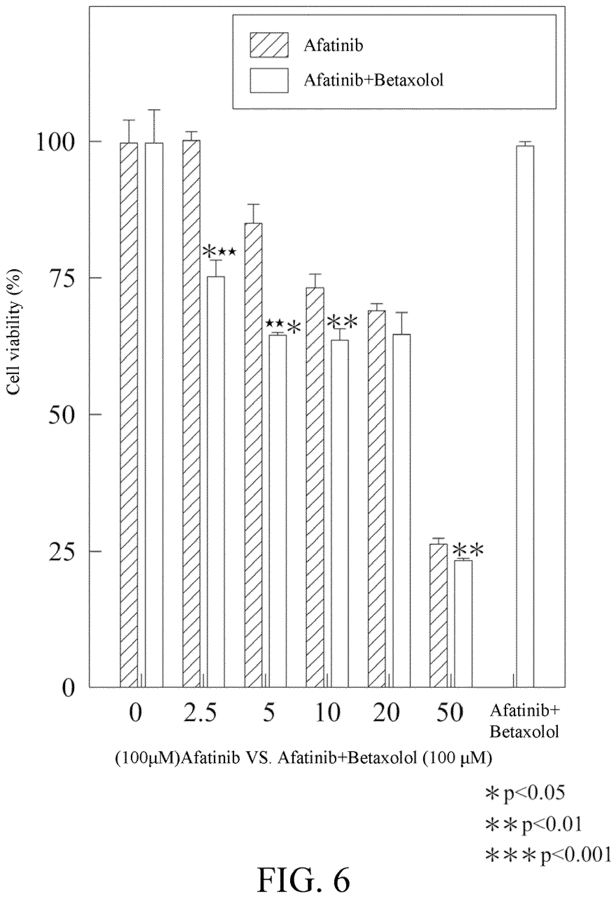

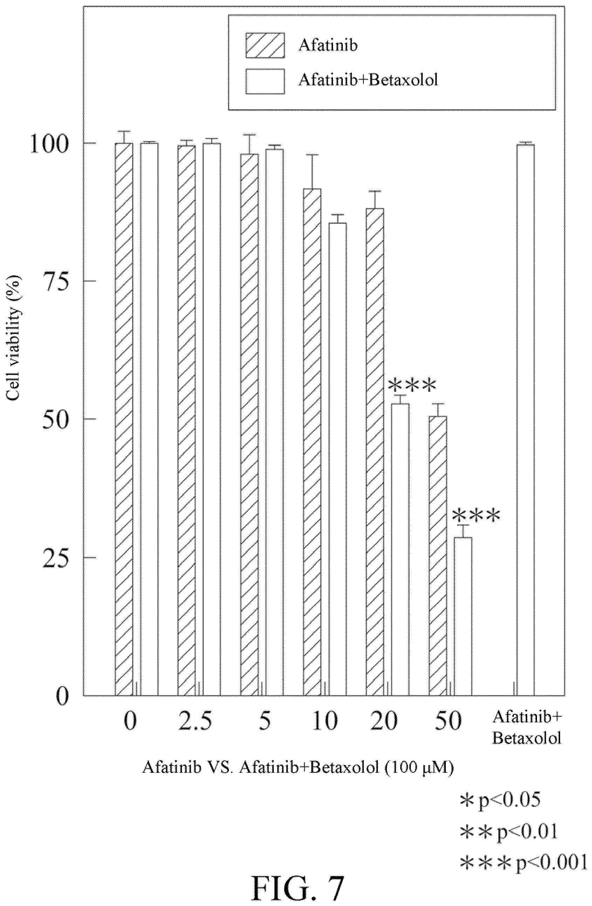

Referring to FIG. 5 to FIG. 7, FIG. 5 is a schematic diagram showing the A549 cancer cell viability after contacting the cancer cells with an EGRF-TKI. FIG. 6 is a schematic diagram showing the A549 cancer cell viability after contacting the cancer cells with an EGFR-TKI only or simultaneously contacting an EGFR-TKI and a β-1 adrenergic receptor antagonist. FIG. 7 is a schematic diagram showing the PC9 cancer cell viability after contacting the cancer cells with an EGFR-TKI only or simultaneously contacting the cancer cells with an EGFR-TKI and a β-1 adrenergic receptor antagonist;

As illustrated in FIG. 5, when afatinib was added to A549 cancer cells (EGFR-mutated lung cancer cells), the cell viability gradually decreased with a higher afatinib concentration. When the afatinib concentration was 50 µM, the A549 cancer cell viability reduced to 26.4%. Therefore, EGFR-TKI effectively increased the apoptosis of cancer cells. The above experiment was repeated 3 times and the absorbance and cell viability data are shown in Table 3 below.

**Table 3**

| Betaxolol | 0µM | DMSO | 1µM | 2.5µM | 5µM | 10µM | 20µM | 50µM |
|---|---|---|---|---|---|---|---|---|
| absorbance (OD₅₇₀) | 0.251 | 0.270 | 0.267 | 0.268 | 0.223 | 0.195 | 0.184 | 0.071 |
| | 0.261 | 0.266 | 0.263 | 0.260 | 0.232 | 0.196 | 0.178 | 0.068 |
| | 0.273 | 0.261 | 0.263 | 0.260 | 0.213 | 0.184 | 0.181 | 0.067 |
| cell viability (%) | 100.0 | 101.6 | 101.1 | 100.4 | 85.2 | 73.4 | 69.1 | 26.4 |

According to FIG. 6, the A549 cancer cell viability was 100% when the cancer cells were only in contact with betaxolol. When A549 cancer cells were in contact with afatinib and betaxolol, the inhibitory effect was better and cytotoxicity is higher than that of afatinib only. Therefore, β-1 adrenergic receptor antagonists can enhance the anticancer effect of EGFR-TKI.

According to FIG. 7, when afatinib and betaxolol are administered simultaneously to PC9 cancer cell (EGFR-mutated cancer cell), the inhibitory effect is better and cytotoxicity is higher than that of afatinib only. Therefore, simultaneously administering β-1 adrenergic receptor antagonist and EGFR-TKI enhances the anticancer effect of EGFR induced cancer cells.

### EXAMPLE 6

In order to verify that only β-1 adrenergic receptor antagonist can reduce epithelial cell damage caused by EGFR-TKI and increase inhibitory effect of EGFR-TKI on cancer cells, anon-selective β-adrenergic receptor antagonist, such as, but not limited to, Timolol, was co-administered with EGFR-TKI. The effects on HaCaT cells as well as A549 cancer cells was examined. The experimental procedure was similar to that of Example 1, and details not repeated here.

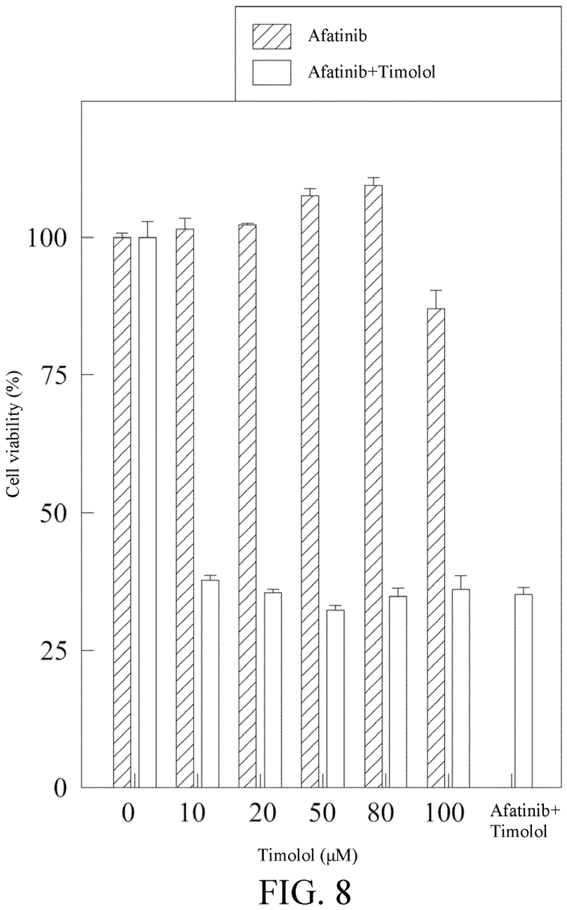

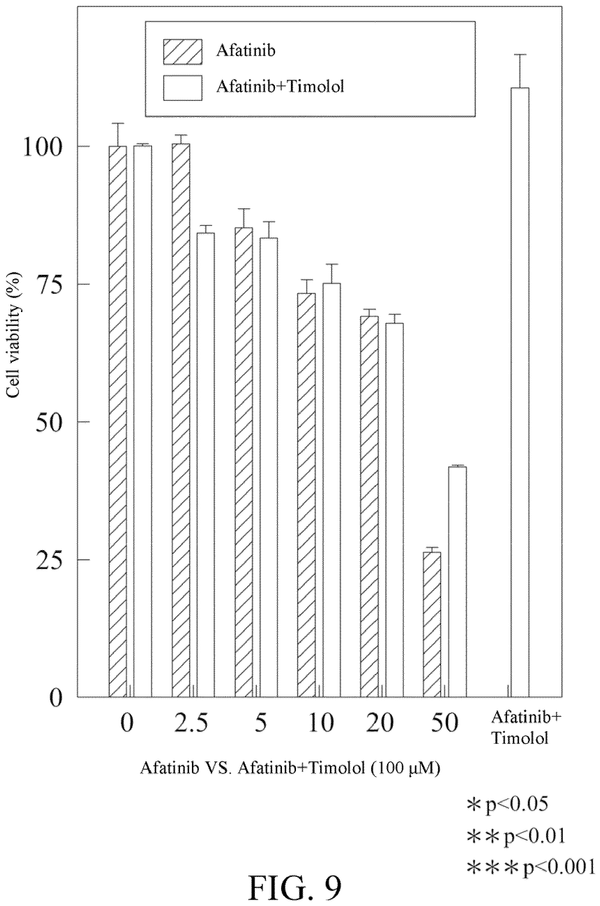

Referring to FIG. 8 and FIG. 9, FIG. 8 is a schematic diagram showing the epithelial cell viability after contacting a non-selective β-adrenergic receptor antagonist for one hour, followed by contacting an EGFR-TKI with normal epithelial cells. FIG. 9 is a schematic diagram showing the A549 cancer cell viability after contacting the cancer cells with an EGFR-TKI and a non-selective β-adrenergic receptor antagonist.

According to FIG. 8, when the concentration of afatinib was fixed and the concentration of timolol was adjusted, the HaCaT cell viability was significantly decreased compared with the HaCaT cells in contact with only afatinib. The cell viability dropped to 37% when HaCaT cells were simultaneously contacted with 10 µM of timolol and afatinib. Therefore, non-selective β-adrenergic receptor antagonists and afatinib significantly reduce the HaCaT cell viability and non-selective β-adrenergic receptor antagonists cannot prevent or reduce damage to normal (or not yet injured) epithelial cells caused by EGFRI cancer treatment.

According to FIG. 9, when timolol and afatinib were simultaneously administered to A549 cancer cells, the A549 cancer cell viability did not change significantly. As the concentration of timolol increased to 50 µM, the A549 cancer cell viability also increased. Therefore, simultaneous administration of a non-selective beta-adrenergic receptor antagonist and an EGFR-TKI does not improve the anticancer effect.

### EXAMPLE 7

Patients with EGFR-TKI or anti-EGFR monoclonal antibodies induced epithelial cell damage, for example, rosacea or papulopustular rash, were recruited. A composition comprising β-1 adrenergic receptor antagonist was formulated for topical use, and was administered to recruited patients to observe its therapeutic effect.

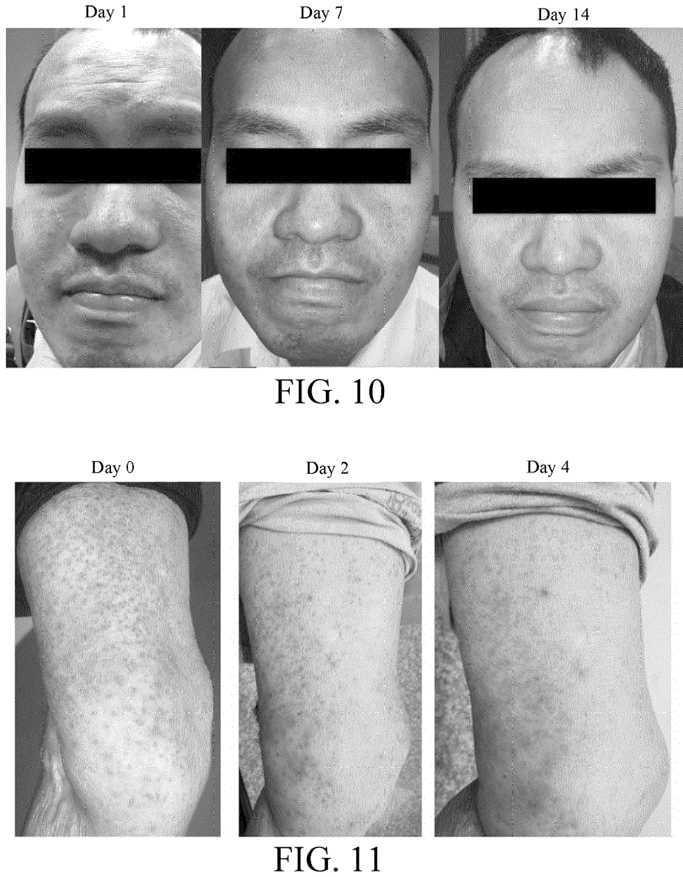

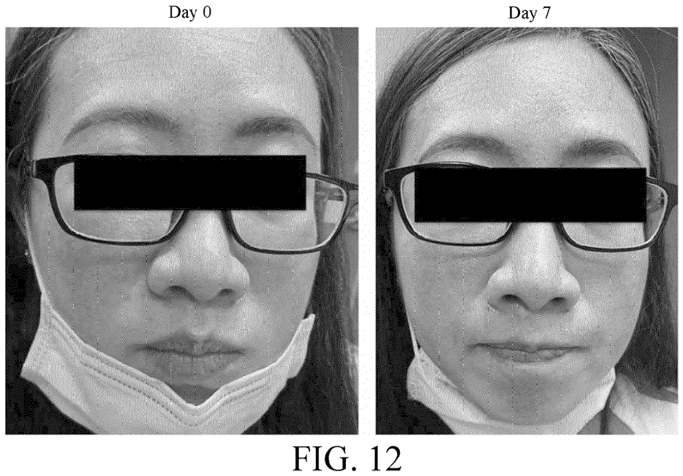

Referring to FIG. 10 to FIG. 12, FIG. 10 to FIG. 12 are diagrams showing the effect of β-1 adrenergic receptor antagonists on EGFRI-induced rosacea. In FIG. 10, 0.25% (w/w) Betaxolol was used to treat afatinib-induced rosacea, and on the 14th day of treatment, rosacea significantly improved compared to the 1st day. FIG. 11 shows the treatment osimertinib-induced rosacea using 0.25% (w/w) betaxolol and rosacea significantly improved on the 4th day of treatment. FIG. 12 shows treatment of rosacea using 0.25% (w/w) betaxolol and rosacea improved significantly on the 7th day of treatment.

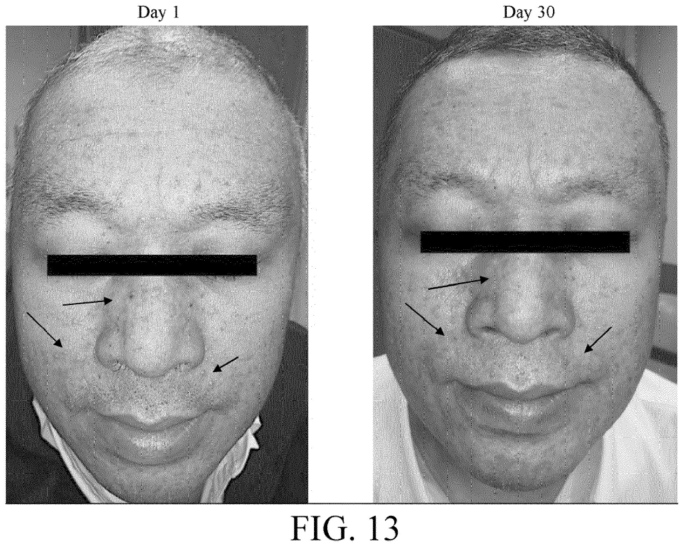

Referring to FIG. 13, FIG. 13 is a schematic diagram illustrating the effect of β-1 adrenergic receptor antagonists on EGFR monoclonal antibody-induced papulopustular rash. 0.25% (w/w) betaxolol was applied to treat papulopustular rash induced by Cetuximab and the papules and pustules (arrows) significantly improved on day 30 of treatment.

In conclusion, β-1 adrenergic receptor antagonists can indeed reduce or prevent EGFR-TKI and anti-EGFR monoclonal antibody (mAb)-induced normal epithelial cell damage, reduce EGFR-TKI and anti-epithelial growth factor receptor monoclonal antibody (mAb)-induced epithelial cell damage, and increase the inhibitory effect of EGFR-TKI on cancer cells. In addition, using non-selective β-adrenergic receptor antagonists as a comparison, the non-selective β-adrenergic receptor antagonists cannot reduce or prevent EGFR-TKI-induced damage to normal epithelial cells, and cannot increase the inhibitory effect of EGFR-TKI on cancer cells.

Therefore, the use of β-1 adrenergic receptor antagonists to reduce EGFR-TKI-induced epithelial cell damage and to improve the inhibitory effect of EGFR-TKI on cancer cells, is unexpected for a person skilled in the art in view of the prior art knowledge

The above-mentioned embodiments merely illustrate the principle and effect of the present invention, but are not intended to limit the present invention. Any person skilled in the art can modify and change the above embodiments without departing from the spirit and scope of the present invention. Therefore, the protection scope of the present invention should be as listed in the claims of the present invention.\\

## Claims

1. Use of aβ-1 adrenergic receptor antagonist for preparation of a composition for reducing or preventing epidermal growth factor receptor inhibitor (EGFRI)-induced damage to normal epithelial cells in a patient, comprising the step of administering to the patient in need thereof the composition comprising an effective amount of β-1 adrenergic receptor antagonist.

2. The use of claim 1, wherein the normal epithelial cells are skin.

3. The use of claim 2, wherein the EGFRI-induced damage to normal skin epithelial cells comprises at least one of the following: papulopustular rash, purpuric drug eruption, skin thinning, dermatitis, rosacea, xerosis, hair thinning, curly hair or skin barrier damage.

4. The use of claim 1, wherein the normal epithelial cells are intestinal cells.

5. The use of claim 4, wherein the EGFRI-induced damage to the normal intestinal epithelial cells comprises at least one of the following: oral mucositis, genital mucositis, or diarrhea.

6. The use of claim 1, wherein the normal epithelial cells are corneal cells.

7. The use of claim 1, wherein the β-1 adrenergic receptor antagonist is selected from the group consisting of atenolol, betaxolol, bisoprolol, esmolol, acebutolol, metoprolol, nebivolol, and any combination thereof.

8. The use of claim 1, wherein the EGFRI is an epidermal growth factor receptor tyrosine kinase inhibitor (EGFR-TKI).

9. Use of a composition comprising an epidermal growth factor receptor tyrosine kinase inhibitor (EGFR-TKI) and a β-1 adrenergic receptor antagonist for manufacturing a medicament for inhibiting cancer cells in a subject, comprising the step of administering to the subject in need thereof of the composition comprising an effective amount of an epidermal growth factor receptor inhibitor (EGFRI) and a β-1 adrenergic receptor antagonist.
